# EUROPEAN PATENT APPLICATION

(11) **EP 4 847 482 A1**
(43) Date of publication of application: **02.12.2026**
(21) Application number: 24920041.1
(22) Date of filing: 09.12.2024
(51) Int. Cl.: C07C 45/49, B01J 23/46, B01J 31/16

(54) **METHOD FOR PRODUCING ALIPHATIC ALDEHYDES**

(30) Priority: 25.01.2024 ES 202430058
(71) Applicant: Consejo Superior de Investigaciones Científicas (CSIC), 28006 Madrid (ES); Universitat Politecnica de Valencia, 46022, Valencia (ES); Max-Planck-Gesellschaft zur Förderung der Wissenschaften e.V., 80539 München (DE)
(72) Inventor: PRIETO GONZÁLEZ, Gonzalo, 46022 Valencia (Valencia) (ES); CETERONI, Ilaria, 46022 Valencia (Valencia) (ES); VORHOLT, Andreas, 80539 München (DE)
(74) Representative: Isern Patentes y Marcas S.L.
(86) International application number: PCT/ES2024/070766
(87) International publication number: WO 2025/158091

(57) **Abstract**

The present invention relates to a method for producing aliphatic aldehydes from syngas, a ruthenium catalyst being used in the process.

## Description

### FIELD OF THE INVENTION

The present invention relates to a method for producing aliphatic aldehydes by means of contacting synthesis gas with a catalyst in a solvent in a reactor. The invention also relates to a catalyst for said method.

### BACKGROUND

Aliphatic aldehydes of general formula RCHO, where R is an alkyl group, are chemical compounds that find applications as intermediates in the production of surfactants and pharmaceutical compounds, in the manufacture of synthetic fragrances and flavours, as polymerisation initiators and cross-linking agents in the manufacture of polymers, or as precursors of other alkyl compounds, such as alcohols and carboxylic acids, among other applications. The term 'fatty aldehydes' is commonly used to refer to aliphatic aldehydes where the alkyl group R contains between approximately 6 and 29 carbon atoms, in other words, they have a hydrocarbon structure Cₙ, where *n* is between approximately 7 and 30 carbon atoms. Linear aliphatic aldehydes with Cₙ alkyl chains of between 8 and 16 carbon atoms, i.e., C₈₋₁₆, such as octanal, nonanal (pelargonic aldehyde), decanal (capric aldehyde), lauric aldehyde (dodecanal), myristaldehyde (tetradecanal) or palmitaldehyde (hexadecanal), are particularly valuable as ingredients in synthetic fragrances and flavours, among other applications.

Methods known in the art for the synthesis of aliphatic aldehydes include the selective dehydrogenation of aliphatic alcohols. Document US5155279A describes a method for preparing an aldehyde which involves reacting the corresponding alkane with a stable free radical nitroxide solubilised as an oxidant. Document WO2019232715A1 discloses a catalyst system for the oxidation of alcohols to aldehydes or ketones using molecular oxygen as the terminal oxidant. The document "An Overview of Selective Oxidation of Alcohols: Catalysts, Oxidants and Reaction Mechanisms." Catal. Surv. Asia 20, 13-22 (2016) provides a review of catalytic methods for the dehydrogenation of alcohols to aldehydes.

An alternative method for the synthesis of aliphatic aldehydes is the hydroformylation of aliphatic alkenes. Hydroformylation, also known as the "oxo" process, involves the reaction of an alkene with synthesis gas, which is a gas mixture comprising carbon monoxide (CO) and hydrogen (H₂), in the presence of a catalyst, adding a formyl group (-CHO) to the initial alkene reactant to form an aldehyde derivative. The document "Hydroformylation: Fundamentals, Processes, and Applications in Organic Synthesis", edited by Armin Bórner and Robert Franke in 2016, ISBN: 9783527335527, provides a review of catalysts and methods for the hydroformylation of alkenes to produce aldehydes. Although rhodium (Rh) is a common active metal in molecular catalysts for the hydroformylation of alkenes, the document by R. Kumar and S.H. Chikkali, "Hydroformylation of olefins by metals other than rhodium", Journal of Organometallic Chemistry, volume 960, 2022, 122231, provides a review of methods for the hydroformylation of alkenes using catalysts based on other metals. Document CN106824282B describes a method for the isomerisation of long-chain internal alkenes and a homogeneous catalytic hydroformylation reaction using rhodium and ruthenium coordination complexes stabilised by phosphine-type ligands as catalysts.

Another method for preparing aliphatic aldehydes is the ozonolysis of aliphatic alkenes, as described for example in the document T. J. Fisher, P. H. Dussault, "Alkene ozonolysis", Tetrahedron, volume 73-30, 2017, pages 4233-4258.

What is common to the above-mentioned methods for preparing aldehydes is that the alkyl hydrocarbon skeleton in the final aldehyde product must be provided in the form of a constituent part (synthon) of a reactant compound, for example, an alcohol, a nitrile, etc. In the case of alkene hydroformylation methods, the Cₙ₋₁ alkyl chain of an alkene reactant is extended by one carbon atom to form a Cₙ aldehyde product. Therefore, although these methods are effective for selectively producing aliphatic aldehydes, they require the supply of a Cₙ or Cₙ₋₁ group, preformed, as part of one of the reactants for the method. This reactant must be produced in another prior method, or be isolated from natural sources. Therefore, it is desirable to develop methods for selectively producing aliphatic aldehydes in which monocarbon compounds (C₁), which are more versatile and cost-effective, are fed as the sole precursors for the main hydrocarbon structure in the aliphatic aldehydes produced.

Synthesis gas, commonly abbreviated as *"syngas",* is a mixture composed of carbon monoxide (CO), hydrogen (H₂) and, in some cases, carbon dioxide (CO ₂) as the main components. Synthesis gas streams may further comprise other gases such as nitrogen (N₂), helium (He), argon (Ar), water vapour (H₂O) or light hydrocarbons such as methane (CH₄), ethane (C₂H₆), propane (C₃H₈), which do not significantly alter the reactivity of the main components. Synthesis gas can be obtained from a wide range of carbonaceous sources, for example, by means of steam reforming or partial oxidation of natural gas or shale gas, coal gasification, biomass gasification and/or reforming, hydrogenation of carbon dioxide or co-electrolysis of CO₂ and water, among others, and is therefore considered a very versatile C₁ precursor for the production of chemical products from a wide range of alternative raw materials to petroleum. Fischer-Tropsch synthesis is a catalytic reaction through which syngas is directly converted into a mixture of synthetic aliphatic hydrocarbons, mainly *n*-paraffins (Fischer-Tropsch Synthesis, Catalysts, and Catalysis: Advances and Applications; B. H. Davis, M. L. Occelli (Eds), CRC Press (2016), ISBN 978-1466555297). Under certain reaction conditions, additionally, the products of the Fischer-Tropsch synthesis may contain oxygenated organic compounds such as aldehydes, alcohols or carboxylic acids. However, in particular, aldehydes are generally obtained only as minor by-products, with selectivities below 5%.

E. Durham, et al. "Diesel-length aldehydes and ketones via supercritical Fischer Tropsch Synthesis on an iron catalyst", Applied Catalysis A: General, volume 386, 1-2, 2010, pages 65-73, and E. Durham, et al. "Supercritical Fischer-Tropsch Synthesis: Heavy Aldehyde Production and the Role of Process Conditions", Industrial & Engineering Chemistry Research 2014 53 (23), 9695-9702, document the production of aldehydes, with a selectivity of up to 30% (on a carbon basis), together with main hydrocarbon products (alkanes and alkenes), in a method for the conversion of synthesis gas (syngas) over a solid iron (Fe) catalyst, which additionally contains copper (Cu) and potassium (K). In this method, the synthesis gas and *n*-hexane are fed to a fixed bed reactor containing the solid catalyst, and the process is carried out at a temperature of 240°C and a total pressure that generates supercritical conditions for *n*-hexane.

A first object of the present invention is to provide a method for producing aliphatic aldehydes by means of converting synthesis gas using a ruthenium catalyst.

### DESCRIPTION

The present invention relates to a method for producing aliphatic aldehydes, which can be implemented by means of at least the following steps:
(i) supplying to a reactor
   a. a synthesis gas feed, and
   b. a solvent
ii) reacting the synthesis gas supplied in step (i) by bringing it into contact with a catalyst comprising ruthenium (Ru), at a reaction temperature comprised between 373 and 523 degrees Kelvin and at a total pressure comprised between 0.1 MPa and 30 MPa, where 1 MPa (megapascal) is equivalent to 10⁶ Pa;
iii) recovering the aliphatic aldehydes from the reaction products of step (ii).

The term "conversion", as it is used in the context of the present invention, is expressed as a percentage and is understood as the fraction of COₓ (x=1 or 2), in the synthesis gas feed which is converted in the reactor to other compounds containing carbon.

The term "selectivity", as it is used in the context of the present invention, is defined on a carbon basis and, therefore, is understood to mean the ratio between the total molar amount of carbon in the products of interest, for example, aliphatic aldehydes, and the total molar amount of carbon in all reaction products, and is expressed on a percentage basis.

The terms "process" and "method", as used interchangeably, in the context of the present invention, are defined as the set of technical operations that involve the development of at least one chemical reaction, and which results in the deliberate modification of at least one chemical compound, which is part of a feed to the method, into at least one different chemical product.

### Method

The types of reactors that are suitable for implementing the method of the invention are those that allow the contact of gaseous and liquid phases with a solid catalyst. In one embodiment of the invention, the reactor operates in continuous mode, whereby at least one gas feed stream and one liquid feed stream are continuously provided to the reactor inlet, such as a slurry bubble column reactor or a trickle-bed reactor, and a product stream is continuously removed at the reactor outlet. In another embodiment of the invention, the reactor operates in batch mode, whereby the gas phase reactants and the liquid phase feeds and the catalyst are introduced into the reactor before the reactor temperature is adjusted to the reaction temperature, the reaction continues without additional streams being provided to the reactor inlet during the reaction. After a certain reaction time, the reaction ends by lowering the reactor temperature below the temperature range for the inventive method, and at least one product stream is collected from the reactor outlet. In another embodiment of the invention, the reactor operates in semi-batch mode, whereby the gas phase reactants and the liquid feeds and the catalyst are introduced into the reactor before the reactor temperature is adjusted to the reaction temperature, the reaction proceeds under the continuous provision of a gas feed stream, for example, synthesis gas, to the reactor inlet. The reaction ends after a certain reaction time by lowering the reactor temperature below the temperature range for the inventive method, and at least one product stream is collected from the reactor outlet.

According to the present invention, the H₂:CO molar ratio in the synthesis gas feed can be in the range of 0.1 to 5.0. In a preferred embodiment of the present invention, said H₂:CO molar ratio in the synthesis gas is in the range of 0.3 to 3.0. More preferably, the H₂:CO molar ratio in the synthesis gas is in the range of 0.5 to 2.0.

According to the present invention, the synthesis gas feed may comprise CO2. The CO₂:CO molar ratio in the synthesis gas feed may be in the range from 0 to 2.0. In a preferred embodiment of the present invention, said CO₂:CO molar ratio in the synthesis gas is in the range of 0 to 0.5. More preferably, the CO₂:CO molar ratio in the synthesis gas is in the range of 0 to 0.1.

The synthesis gas feed can also comprise other compounds that do not interfere with the conversion of the synthesis gas, such as nitrogen, helium, argon, water, C₁-C₄ alkanes (alkanes comprising between 1 and 4 carbon atoms) or combinations thereof.

The solvent provided in step (i) provides better temperature control and a means of solubilising compounds in the reactor. Said compounds comprise products of the synthesis gas conversion reaction, ligands, metal species of the catalyst or any combination thereof. Said solvent can be selected from the list of aliphatic hydrocarbons, aromatic hydrocarbons, oxygenated organic compounds, water, nitrogenous organic compounds, halogenated organic compounds, liquid organic salts and combinations thereof. More specifically, aliphatic hydrocarbon solvents can be selected from the non-limiting list consisting of *n-hexane*, *n*-decane, *n*-dodecane, *n*-hexadecane, *iso*-pentane (2-methylbutane), *iso*-hexane (2-methylpentane), *iso*-octane (2,2,4-trimethylpentane) and 2,6,11,15-tetramethylhexadecane. Again, more specifically, aromatic hydrocarbon solvents can be selected from the non-limiting list consisting of benzene, toluene, xylene, trimethylbenzenes and naphthalene. Again, more specifically, the oxygenated organic compound solvents can be selected from the non-limiting list consisting of methanol, ethanol, *iso*propanol, *n*-butanol, *n-*hexanol, 1,4-dioxane, dimethylformamide, tetrahydrofuran, di-*n*-butyl ether, cyclohexanone, methyl isobutyl ketone, ethylene glycol, propylene glycol, cyclopropyl carbonate, glycerol and ethyl acetate. Again, more specifically, the nitrogenous organic compound solvents can be selected from the non-limiting list consisting of acetonitrile, benzonitrile, N,N-dimethylaniline, N-methylmorpholine, N-methylpiperidine, N-methylpyrrole and quinoline. Again, more specifically, the halogenated organic compound solvents can be selected from the non-limiting list consisting of methylene chloride, chloroform, 1,2-dichloroethane, perfluoropentane, perfluorohexane, perfluorotoluene, monofluorobenzene and perfluorobenzene. Again, more specifically, the liquid organic salt solvents are selected from the non-limiting list consisting of salts comprising organic cations of the imidazolium, pyridinium, 1,2,3-triazolium, morpholinium, piperidinium, pyrrolidinium, pyrazolium, ammonium and phosphonium type, associated with anions of the PF⁶⁻, BF⁴⁻, NTf²⁻ (bis(trifluoromethylsulfonyl)amide), OTf⁻ (trifluoromethylsulfonate), DCA⁻ (dicyanamide), acetate and CH₃SO₃⁻type. In another particular embodiment of the invention, the solvent comprises aliphatic aldehydes of the type produced in the reaction stage of the method of the invention.

According to the present invention, the method is carried out at a reaction temperature in the range of 373 degrees Kelvin (K) to 523 K. The reaction temperature, as it is used in the context of the present invention, is understood as the maximum temperature in the reactor measured by means of a type K thermocouple located inside a stainless steel sheath and in contact with the liquid phase. In a preferred embodiment of the present invention, the method is carried out at a reaction temperature in the range of 393 K to 453 K. More preferably, the method is carried out at a reaction temperature in the range of 413 K to 433 K.

The suitable operating pressures for the method of the invention are between approximately 0.1 MPa and 30 MPa. In a preferred embodiment of the present invention, the method is carried out at a pressure of between 1 MPa and 20 MPa. In an even more preferred embodiment of the present invention, the method is carried out at a reaction pressure of between 4 MPa and 10 MPa.

In the method of the invention, the distribution of aliphatic aldehyde products may obey what is known as the Anderson-Schulz-Flory (ASF) chain length distribution, which is typically observed for reactions that proceed through a chain growth polymerisation mechanism from C₁ units. This product distribution is described mathematically with the following relationship (equation 1): ${W}_{n} = n ⋅ {\left(1-α\right)}^{2} ⋅ {α}^{n - 1}$ (Equation 1) in which Wₙ is the mass fraction of the aliphatic aldehyde products (RCHO) for which the hydrocarbon chain R contains *n* carbon atoms, and α (alpha) represents the probability factor of chain growth, which represents the probability, on a basis of 0 to 1, that in the polymerisation process a C₁ monomer species is added to a hydrocarbon chain, so that the latter grows by one carbon atom. Experimentally, the probability of chain growth (alpha) can be determined according to the relationship *α=e ^{m},* where e is the Euler number and m represents the slope obtained for the mathematical function of linear regression of the data in a graph representing Ln(W*ₙ*/n) versus *n* over a sufficiently wide range of carbon chain lengths *n,* in which Ln represents the natural logarithmic function.

Normally, the mixture of products from the inventive method is fractionated to recover aldehyde products. The recovery of the aldehyde products from mixtures with other reaction products for example paraffins, can be carried out by means of known methods such as distillation, liquid-liquid extraction, selective adsorption or reactive derivatisation of aldehydes to other derivative compounds, such as hydrazones, oximes or organoboron compounds, followed by any of the previous methods.

Preferably, in the method of the present invention, linear aliphatic aldehydes with a hydrocarbon chain length of between 8 and 16 carbon atoms are produced. In a particular embodiment of the invention, the compounds present in the reactor effluent stream, such as reactants that have not been converted, solvent, reaction products, or a combination thereof, are recovered and recirculated to the reactor.

### Catalyst

According to the present invention, the catalyst is a solid. In a particular embodiment of the invention, the catalyst is a supported metal catalyst. The concept of "supported metal catalyst" is understood in the art as a catalyst composition comprising, on the one hand, a metal part, dispersed in the form of particles with an average diameter typically less than 100 nm, which is generally catalytically active, or can become an active phase *in situ,* before or during the use of the catalyst, and, on the other hand, a non-metal part, the carrier or support material, which is generally porous and forms most of the catalyst by mass.

Various methods are known in the art for incorporating a metal part into a support material. These methods include preparation techniques such as impregnation, precipitation by deposition, ion exchange, electrochemical deposition, electrostatic adsorption, fusion infiltration and co-precipitation. The documents "Handbook of Heterogeneous Catalysis", G. Ertl, H. Knözinger, F. Schüth, J. Weitkamp (Editors); volume 1, Wiley-VCH Verlag GmbH & Co. Weinheim, 2008 and "Synthesis of solid catalysts", K. P. de Jong (Editor); Wiley-VCH Verlag GmbH & Co. Weinheim, 2009 provide references to existing methods for incorporating a metal part into a support material.

According to the present invention, the solid catalyst comprises ruthenium (Ru) as the active metal. According to a preferred embodiment of the invention, Ru represents between 50% and 100% of the metal part. More preferably, Ru represents between 80% and 100% of the metal part.

The support material must be chemically stable under the reaction conditions found in a synthesis gas conversion method. Said support can be an oxide selected from the porous oxide-type supports used as support materials for supported metal catalysts in the art, and which can comprise among others, SiO₂, transition forms of AI₂O₃, α-AI₂O₃, TiO₂, ZrO₂, carbide or oxycarbide material such as SiC, SiOₓC_{y}, with x in the range of 0<x<2 and y in the range of 0<y<1, carbon and any combination thereof. Transition forms of AI₂O₃ are understood to be metastable polymorphs of aluminium (III) oxide that can be obtained at intermediate temperatures during the transformation of aluminium hydroxides and oxohydroxides to the most thermodynamically stable phase, this is alpha-Al₂O₃ (α-AI₂O₃). Said transition forms of AI₂O₃ include chi-Al₂O₃, kappa-Al₂O₃, gamma-Al₂O₃, delta-Al₂O₃, theta-Al₂O₃, eta-Al₂O₃, rho-Al₂O₃, and combinations thereof.

Preferably, the loading by weight of the metal, preferably Ru, defined as the fraction by weight of the total catalyst corresponding to the metal, preferably Ru, in said catalyst, is in the range of 0.5 - 50% by weight, and more preferably in the range of 3 - 20% by weight.

The metals are incorporated onto the support material using metal precursor compounds such as inorganic and organic metal salts, metal groups, and organometallic complexes. Examples of precursors containing ruthenium are ruthenium acetylacetonate, ruthenium chloride, ruthenium nitrosyl nitrate, ammonium hexachlororuthenate, triruthenium dodecacarbonyl, bis(cyclopentadienyl)ruthenium(II) and the like. The ruthenium precursors can provide ruthenium in a zero oxidation state, oxidation state II, oxidation state III, oxidation state IV, oxidation state VI, or a combination thereof. According to a preferred embodiment of the invention, the catalyst is prepared using ruthenium nitrosyl nitrate as the ruthenium precursor. Generally, after the incorporation of the metal precursor, a heat treatment is applied under a gas atmosphere, sealed or flowing, of air, water vapour, an inert gas such as nitrogen, helium, argon or combinations thereof, to decompose the metal precursor into an oxide or suboxide form on the surface of the support material.

In another particular embodiment of the invention, the catalyst is an unsupported (bulk) metal catalyst, such as a finely divided metal powder, a metal gauze or a metal foam.

In a particular embodiment of the invention, the catalyst comprises, in addition to Ru, also a second metal (M2) selected from the list of rhodium (Rh), cobalt (Co), iridium (Ir), and any combination thereof. In a preferred embodiment, the catalyst comprises, in addition to Ru, rhodium (Rh). The content of the second metal M2, expressed as the M2:Ru molar ratio is preferably comprised between 0.001 and 1, more preferably between 0.001 and 0.5, and even more preferably between 0.001 and 0.2.

It is common in the art for supported metal catalysts to comprise what are known as promoters, in addition to the main active metal and the support material in the case of supported catalysts. Generally, a promoter is a substance that improves the performance of the catalyst in terms of activity, selectivity to a desired product or fraction of products, for example, aliphatic aldehydes in the present invention, stability under operating conditions, or a combination thereof.

In another particular embodiment of the invention, the catalyst comprises at least one promoter. Said promoter can be selected from the list of alkali metals, alkaline earth metals, lanthanides, transition metals (other than ruthenium (Ru), rhodium (Rh), iridium (Ir) and cobalt (Co)), boron (B), aluminium (Al), gallium (Ga), indium (In), carbon (C), germanium (Ge), tin (Sn), nitrogen (N), phosphorus (P), arsenic (Ar), antimony (Sb), and any combination thereof. Any of these elements may be in elemental form or in ionic form. In a preferred embodiment of the invention, the promoter contributes to greater selectivity to aliphatic aldehydes. In a preferred embodiment of the invention, said promoter is selected from the list of alkali metals, alkaline earth metals, lanthanides, yttrium (Y), manganese (Mn), zinc (Zn), gallium (Ga), boron (B), indium (In) and any combination thereof. In an even more preferred embodiment of the invention, said promoter is selected from the list of alkali metals, alkaline earth metals, lanthanides, Y, B, Mn and any combination thereof. Said promoter may be present in a content in the range of 0.001 - 50% by weight, preferably in the range of 0.001 - 20% by weight, and more preferably in the range of 0.001 - 10% by weight, calculated on the basis of the total mass of the promoter element to the total mass of the catalyst.

Suitable methods for incorporating the second metal M2 and/or promoter elements include techniques known in the art such as impregnation, precipitation by deposition, ion exchange, electrochemical deposition, electrostatic adsorption, fusion infiltration or co-precipitation with suitable precursors, including inorganic and organic salts, the metal groups and organometallic complexes of the corresponding element. Examples of precursors containing rhodium are rhodium acetylacetonate, rhodium chloride, rhodium nitrate, rhodium sulphate, dirhodium carbonyl chloride, rhodium (II) acetate dimer, dicarbonyl (pentamethylcyclopentadienyl) rhodium (I), acetylacetone (1,5-cyclooctadiene) rhodium (I) and the like. The rhodium precursors can provide ruthenium in a zero oxidation state, oxidation state I, oxidation state II, oxidation state III, or a combination thereof.

Before the application thereof in the method of the invention, either with or without a support, the catalyst is normally subjected to heat treatments in the presence of hydrogen or an alternative reducing agent to convert all or part of the active metal part to the metallic state, in other words, zerovalent. In a preferred embodiment, said reduction treatment takes place at a temperature in the range of 373-873 K, and more preferably in the range of 373-773 K in the flow of a stream comprising hydrogen, either as a pure gas or in combination with an inert gas selected from the list of nitrogen, helium, argon or combinations thereof. Said heat treatment may be omitted in cases where exposure to the reaction conditions of the method of the invention, in other words, in the presence of synthesis gas as a reducing atmosphere, is sufficient to convert all or part of the active metal part to a metallic state, *in situ,* in the reactor of the method of the invention.

During step (ii) of the method of the invention, the metal of the catalyst, that is ruthenium, alone or combined with a second metal (M2) selected from the list of rhodium (Rh), cobalt (Co), iridium (Ir), and any combination thereof, can have one or more formal oxidation states in the reactor. Said metal preferably has an oxidation state of +3 or lower, more preferably an oxidation state of +2 or lower, even more preferably an oxidation state of +1 or lower, and even more preferably an oxidation state of 0, in other words, a zerovalent state.

In a particular embodiment of the invention, the catalyst is added to the reaction medium in a ratio of catalyst mass to solvent volume in the range of 0.001 kg/l to 0.1 kg/l, preferably in the range of 0.001 kg/l to 0.01 kg/l.

In a particular embodiment of the invention, an organic ligand is added to the liquid phase in which the catalyst is suspended in the solvent. The term "ligand", as it is used in the context of the present invention, is understood as an ion or organic molecule that can bind to a metal by means of coordinative bonds, giving rise to a coordination complex. The role of the ligand is to promote the activity of the catalyst towards greater selectivity to aliphatic aldehydes. Without wishing to be bound by any particular theory, it is considered that the ligand can form a coordination complex with one or more metal elements of the catalyst, which could have a chemical formula of the type Hₓ(M)_{y}(CO)_{z}(L)ₙ, where *x* is equal to or greater than zero, *y* is equal to or greater than 1, z is equal to or greater than zero and *n* is equal to or greater than zero, and M denotes a metal, and L denotes an organic ligand.

The organic ligand can be selected from the list including ligands containing phosphorus, ligands containing nitrogen, containing oxygen, and combinations thereof. In particular, the ligands containing phosphorus may be selected from the list including phosphines, polyphosphines, phosphine oxides and phosphites. Non-limiting examples include triethylphosphine, tributylphosphine, trihexylphosphine, trioctylphosphine, triisopropylphosphine, tricyclohexylphosphine, tricyclopentylphosphine, hexylphosphine, cyclohexylphobane, triphenylphosphine, diethylphenylphosphine, ethyldiphenylphosphine, bis(diphenylphosphino)ethane, bis(diphenylphosphino)butane, triphenyl phosphite, 6,6'-[(3,3'-Di-*tert*-butyl-5,5'-dimethoxy-[1,1'-biphenyl]-2,2'-diyl)bis(oxy)]bis(6H-dibenzo[d,f][1,3,2]dioxaphosphepine *(Biphephos),* (9,9-Dimethyl-9H-xanthene-4,5-diyl)bis-diphenylphosphane *(Xantphos),* 1,2-bis(diphenylphosphino)ethane *(dppe),* 2-(Dicyclohexylphosphino)-1-methyl-1H-imidazole, and combinations thereof. In particular, the ligands containing nitrogen may be selected from the list including monoamines, diamines, triamines and polyamines, imines, diimines, pyridines, bipyridines, imidazoles, pyrroles and pyrazoles. In particular, ligands containing oxygen may be selected from the list including alcohols, ethers, ketones, acetals and combinations thereof. In a preferred embodiment of the invention, the ligand is selected from the list including ligands containing phosphorus.

In a particular embodiment of the invention, the ligand is added to the reactor in a ligand:M molar ratio, defined as the ratio of the moles of ligand to the moles of metal M, comprised between 0.1 and 100, preferably between 0.1 and 10 and more preferably between 0.1 and 1.0, where M represents Ru, a second metal (M2) selected from the list of rhodium (Rh), cobalt (Co), iridium (Ir), and any combination thereof in the catalyst formulation.

The present invention is described in more detail by means of the following figures and experimental examples.

### FIGURES

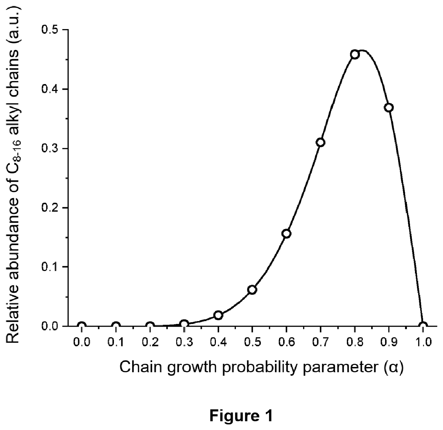

**Figure 1****:** evolution of the relative abundance of products with hydrocarbon chain lengths in the range of C₈₋₁₆, along the y-axis, in arbitrary units, increasing from bottom to top, as a function of the chain growth probability parameter (dimensionless) along the x-axis, increasing from left to right, according to an Anderson-Schulz-Flory (ASF) distribution. The line joining the scattered data points corresponds to the interpolated *spline* function.

### EXAMPLES

The following examples are provided by way of illustration and are not intended to limit the scope of the invention.

The following experimental methods have been used to determine the properties of the materials prepared and used in the following examples:
(i) The specific surface area and pore volume of the support materials used for the synthesis of catalysts were determined by means of nitrogen adsorption. The nitrogen adsorption isotherms were recorded using an ASAP 2420 apparatus (Micromeritics) at -196°C (77 K). Before analysis, approximately 200 mg of the sample (0.2 - 0.4 mm sieve fraction) was degassed at a temperature of 673 K and a vacuum of ~5x10 ⁻¹ Pa for 15 hours. The specific surface area was determined by applying the Brunauer-Emmett-Teller (BET) equation in the relative pressure range (P/P°) of 0.05 - 0.3. The pore volume was derived from nitrogen adsorption at a relative pressure P/P ⁰=0.95.
(ii) The elemental composition of the catalysts was determined by means of X-ray fluorescence spectroscopy (XRF) on a Zetium 4 kV spectrometer (Malvern Panalytical), equipped with an Rh cathode as an X-ray source. The measurements of the powder samples were taken at room temperature and under helium atmosphere. Before the measurements, the device was calibrated by preparing solid mixtures containing known metal concentrations, within the range consistent with the theoretical metal content of the catalysts. The calibration was repeated for each support material.

### Synthesis of catalysts according to the invention

### Ru/SiO₂ catalyst

A ruthenium precursor solution was prepared by dissolving ruthenium (III) nitrosyl nitrate (N₄O₁₀Ru, Alpha Aesar, Ru 31.3% minimum content) in a 0.1 M HNO₃ solution in deionised water. Specifically, 1.0 g of metal precursor was added to 2.5 ml of acid solution. Silica gel (S10020M - Silicycle) powder was used as the catalyst support. The metal was incorporated by means of incipient wetness impregnation. The SiO₂ support was first dried in a glass flask at 423 K for 15 hours under a dynamic vacuum (1.5 mbar). Next, a volume of ruthenium precursor solution, equivalent to 90% of the pore volume of the SiO₂ support (0.72 cm³/g), with the dry support, under magnetic stirring and static vacuum. After the impregnation, the material was kept under dynamic vacuum (1.5 mbar) for at least 3 h, it was transferred to a quartz fixed-bed tubular reactor, axially mounted in a furnace, and was treated at 623 K for 3 h (heating ramp at room temperature of 1 K/min) under a flow of N₂/H₂ (80% N₂, 20% H₂, vol) to decompose the Ru precursor and convert the metal to its metallic state. After the heat treatment, the tubular reactor was purged with N₂ flow and the activated catalyst was transferred to a glove box filled with N₂ (O₂<1.0 ppm, H₂O<0.1 ppm) for storage. The Ru content in the Ru/SiO₂ catalyst was 7.0% by weight.

### Ru/Al₂O₃ catalyst

The Ru/AI₂O₃ catalyst was synthesised as detailed above for Ru/SiO₂, using γ-AI₂O₃ as the support material in this case. The γ-AI₂O₃ support was synthesised by means of calcination of a pseudo-boehmite precursor (DISPERAL 80, Sasol) at 823 K (heating ramp of 2 K/min from room temperature) for 5 h in a muffle furnace under an air atmosphere, and presented a pore volume of 0.66 cm³/g. After the incorporation of Ru by incipient wetness impregnation, as detailed above, the solid was dried at room temperature under a dynamic vacuum (1.5 mbar) for at least 3 h, it was transferred to a quartz fixed-bed tubular reactor, axially mounted in a furnace, and was treated at 623 K for 3 h (heating ramp from room temperature of 1 K/min) under an N₂/H₂ flow (80% N₂, 20% H₂, vol) to decompose the Ru precursor and convert the metal to its metallic state. The Ru content in the Ru/AI₂O₃ catalyst was 8.0% by weight.

### General experimental method for testing catalysts according to the invention

In a general experimental method, the conversion reaction is carried out in a 316L stainless steel batch reactor (ILSHIN), magnetically stirred, with an internal volume of approximately 30 ml. The temperature reading is taken with a type K thermocouple covered with a 316L stainless steel sheath, inserted into the liquid reaction medium, while the pressure reading is performed by means of a digital pressure transducer (Sensys).

Before the reaction tests, all liquid reactants were dried for 15 hours using a thermally activated molecular sieve (5 Å, particle size 3.2 mm, Sigma Aldrich) and then stored in a glove box with N₂ atmosphere (O₂ < 1.0 ppm, H₂O <0.1 ppm). Also under exclusion of air, the powdered catalyst, the solvent, optionally the organic ligand, and a magnetic stirring bar (L x D 9 mm x 20 mm) were added to a cylindrical PTFE liner, which was capped and inserted into the body of the autoclave reactor. Next, the autoclave was sealed and purged three times with a synthetic synthesis gas mixture containing CO/H₂/Ar (Ar used as internal standard for gas chromatography analysis of gaseous products) in pre-set volumetric proportions, and then filled with the same synthesis gas mixture at a pre-set total pressure P (bar) at room temperature. The reactor was inserted into a custom-made aluminium block mounted on a stirring/heating plate (Heidolph), the stirring speed was adjusted to 800 rpm and the temperature inside the autoclave liner was raised to the reaction temperature at a rate of 3 K/min. After a selected reaction time, the autoclave reactor was cooled to room temperature. The gaseous phase in the headspace was sampled on an Agilent 7890 gas chromatograph equipped with two analysis channels and using He as the carrier gas. A first channel with a HP-PLOT-Q compact column (30 m, 0.32 mm internal diameter, 20.0 µm film thickness) and an HP-MOLSIEVE compact column molecular sieve (30 m, 0.32 mm internal diameter, 12.0 µm film thickness) and a TCD detector for the analysis of permanent gases and carbon dioxide using argon as an internal standard; and a second analysis channel equipped with a DB1 capillary column (60 m, 0.32 mm internal diameter, 3 µm film thickness) and an FID detector for the analysis of hydrocarbons and oxygenated organic compounds. The liquid phase of the product was collected directly from the liner inside the reactor and filtered to remove solid particles prior to analysis. The gas chromatography analysis was performed on a Shimadzu GC-2010 Plus gas chromatograph equipped with an HP 5 capillary column (30 m, 0.25 mm internal diameter, 0.25 µm film thickness) and an FID detector, using H₂ as the carrier gas and tetrahydrofuran (THF) as the external standard. Both liquid and gaseous phase products were identified by means of gas chromatography/mass spectrometry using an Agilent 8890 gas chromatograph paired with a 5977B single quadrupole GC/MSD equipped with an HP5 capillary column (30 m, 0.32 mm internal diameter, 0.32 µm film thickness) for compound separation and an FID detector for analysis. Helium was used as the carrier gas. The mass-based response factors in the FID detector of those reaction products not available in pure form from commercial suppliers were determined using a poIyARC microreactor coupled with the FID detector in the Agilent 8890 gas chromatograph.

### Example I

In an example according to the present invention, 148 mg of Ru/AI₂O₃ was used as catalyst and 12 ml of 1,4-dioxane (Sigma Aldrich, 99.8%) was added to the reactor as solvent. The experiment was performed according to the general experimental method for catalytic tests described above. The H₂:CO molar ratio in the feed synthesis gas was 1:1. The reaction time was 24 h.

### Example II

In an example according to the present invention, 267 mg of Ru/AI₂O₃ was used as catalyst and 12 ml of iso-octane (Sigma Aldrich, 99.8%) was added to the reactor as solvent. The experiment was performed according to the general experimental method for catalytic tests described above. The H₂:CO molar ratio in the feed gas was 2:1. The reaction time was 24 h.

### Example III

In an example according to the present invention, 182 mg of Ru/SiO₂ was used as catalyst and 12 ml of iso-octane (Sigma Aldrich, 99.8%) was added as solvent. The experiment was performed according to the general experimental method for catalytic tests described above. The H₂:CO molar ratio in the feed gas was 1:1. The reaction time was 24 h.

### Example IV

In an example according to the present invention, 180 mg of Ru/SiO₂ was used as catalyst and 12 ml of iso-octane (Sigma Aldrich, 99.8%) was added to the reactor as solvent. The experiment was performed according to the general experimental method for catalytic tests described above. The H₂:CO molar ratio in the feed gas was 1:1. The reaction time was 24 h.

### Example V

In an example according to the present invention, 177 mg of Ru/SiO₂ was used as catalyst and 12 ml of iso-octane (Sigma Aldrich, 99.8%) was added to the reactor as solvent. The experiment was performed according to the general experimental method for catalytic tests described above. The H₂:CO molar ratio in the feed gas was 1:1. The reaction time was 24 h.

### Example VI

In an example according to the present invention, 145 mg of Ru/SiO₂ was applied as catalyst, and 12 ml of iso-octane (Sigma Aldrich, anhydride, 99.8%) was added as solvent. The method was performed according to the general experimental method for catalytic tests described above. The H₂:CO molar ratio in the feed gas was 1.5:1. The reaction time was 24 h.

### Example VIl

In an example according to the present invention, 231 mg of Ru/SiO₂ was used as catalyst and 12 ml of iso-octane (Sigma Aldrich, anhydride, 99.8%) was added as solvent. The experiment was performed according to the general experimental method for catalytic tests described above. The H₂:CO molar ratio in the feed gas was 1:1. The reaction time was 24 h.

### Example VIII

In an example according to the present invention, 181 mg of Ru/AI₂O₃ was used as catalyst, 12 ml of iso-octane (Sigma Aldrich, anhydride, 99.8%) was added as solvent and 2.5 mg of triphenylphosphine (Sigma Aldrich, 99.9%) as organic ligand. The experiment was performed according to the general experimental method for catalytic tests described above. The H₂:CO molar ratio in the feed gas was 1:1. The reaction time was 24 h.

**Table 1: Summary of conversion and product selectivities for the examples carried out according to the method of the invention.**

| **Example** | **T^{(a)} (K)** | **p(b) (MPa)** | **CO:M^{(c)} (-)** | **Xco^{(d)} (%)** | **Selectivity (€ ^{(e)}** | | | | **α^{(g)} (-)** |
|---|---|---|---|---|---|---|---|---|---|
| | | | | | **CO₂** | **Hydrocarbons^{(f)}** | **Aliphatic aldehydes** | **Aliphatic alcohols** | |
| I | 433 | 3.7 | 70:1 | 33.3 | 0.5 | 63.0 | 29.0 | 7.5 | 0.83 |
| II | 413 | 7.5 | 70:1 | 28.4 | 0.6 | 59.1 | 40.3 | n.d. | 0.82 |
| III | 433 | 3.7 | 70:1 | 42.6 | 6.2 | 51.8 | 42.0 | n.d. | 0.83 |
| IV | 453 | 3.9 | 70:1 | 48.3 | 7.3 | 58.3 | 34.4 | n.d. | 0.85 |
| V | 473 | 4.5 | 70:1 | 52.1 | 10.0 | 56.0 | 34.0 | n.d. | 0.83 |
| VI | 433 | 4.5 | 80:1 | 73.9 | 2.8 | 61.3 | 35.9 | n.d. | 0.85 |
| VII | 433 | 7.2 | 100:1 | 38.2 | 3.4 | 57.2 | 39.4 | n.d. | 0.81 |
| VIII | 433 | 3.8 | 70:1 | 32.6 | 7.0 | 55.2 | 37.8 | n.d. | 0.81 |

| | | | | | | | | | |
|---|---|---|---|---|---|---|---|---|---|
| (a) Reaction temperature in degrees Kelvin; (b) Total pressure, in megapascals, determined at the beginning of the reaction and at the reaction temperature; (c) Molar ratio of CO in the synthesis gas feed to metal in the catalyst; (d) Conversion of CO; (e) Selectivities expressed on a carbon molar basis; (f) Non-oxygenated hydrocarbons; (g) Chain growth probability parameter determined from the analysis of the alkyl chain length in the aliphatic aldehyde products according to an ASF distribution considering compounds in the chain length range n=C₅-C₂₅. n.d.: not detected. | | | | | | | | | |

Although the present invention has been described in terms of preferred embodiments, it is understood that said description is not to be construed as a limitation of the invention described herein. Upon reading the description, it will be immediately apparent to those with ordinary skill in the field, in view of the teachings of this invention, that various alterations and modifications can be made to it. The appended claims are to be construed as encompassing all alterations and modifications falling within the spirit and scope of the present invention.

## Claims

1. A method for producing aliphatic aldehydes comprising at least the following steps:
(i) supplying to a reactor
a. a synthesis gas feed, and
b. a solvent
ii) reacting the synthesis gas supplied in step (i) over a catalyst comprising ruthenium (Ru), either in unsupported form or dispersed over a support material, at a reaction temperature comprised between 373 and 523 degrees Kelvin (K) and at a total pressure comprised between 0.1 MPa and 30 MPa;
iii) recovering the aliphatic aldehydes from the reaction products of step (ii).

2. The method according to claim 1, in which the catalyst is a supported catalyst in which Ru is dispersed over a support material selected from the list comprising: SiO₂, transition forms of AI₂O₃, C₁-AI₂O₃, TiO₂, ZrO₂, carbide or oxycarbide materials selected from: SiC and SiOₓC_{y}, x being in the range of 0<x<2 and y being in the range of 0<y<1, carbon and any combination thereof.

3. The method according to any of the preceding claims, in which the weight fraction of the total catalyst corresponding to Ru is in the range of 0.5 - 50% by weight.

4. The method according to any of the preceding claims, in which the catalyst also comprises a second metal M2 selected from the list of rhodium (Rh), cobalt (Co), iridium (Ir) and any combination thereof.

5. The method according to any of the preceding claims, in which the content of the second metal (M2), expressed as the M2:Ru molar ratio, is comprised between 0.001 and 1.

6. The method according to any of the preceding claims, in which the catalyst also comprises a promoter element selected from the list comprising: alkali metals, alkaline earth metals, lanthanides, transition metals other than ruthenium (Ru), rhodium (Rh), iridium (Ir) and cobalt (Co), boron (B), aluminium (Al), gallium (Ga), indium (In), carbon (C), germanium (Ge), tin (Sn), nitrogen (N), phosphorus (P), arsenic (Ar), antimony (Sb), and any combination thereof.

7. The method according to claim 6, in which the promoter element is present in a content of between 0.001 and 50% by weight, calculated on the basis of the total mass of the promoter element with respect to the total mass of the catalyst.

8. The method according to any of the preceding claims, in which the solvent is selected from the list comprising: aliphatic hydrocarbons, aromatic hydrocarbons, oxygenated organic compounds, water, nitrogenous organic compounds, halogenated organic compounds, liquid organic salts and combinations thereof.

9. The method according to any of the preceding claims, in which to the solvent is also added an organic ligand selected from the list comprising: ligands containing phosphorus, ligands containing nitrogen, ligands containing oxygen, and combinations thereof.

10. The method according to claim 9, in which the organic ligand is selected from the list including phosphines, polyphosphines, phosphine oxides and phosphites.

11. The method according to any of the preceding claims, in which the reaction is carried out in a stirred batch reactor.

12. The method according to any of claims 1 to 10, in which the reaction is carried out in a continuous reactor to which both a gas stream and a liquid stream are fed.

13. The method according to any of the preceding claims, in which the reaction temperature is in the range of between 393 K and 453 K.

14. The method according to any of the preceding claims, in which the reaction pressure is in the range of 1 MPa to 20 MPa.

15. The method according to any of the preceding claims, in which the H₂:CO molar ratio in the synthesis gas feed is between 0.1 and 5.0.

16. The method according to any of the preceding claims, where the aliphatic aldehydes are linear aliphatic aldehydes with a hydrocarbon chain length of between 8 and 16 carbon atoms.

17. The method according to any of the preceding claims, in which the synthesis gas feed additionally comprises other compounds such as nitrogen, helium, argon, water, C₁-C₄ alkanes or combinations thereof.

18. The method according to any of the preceding claims, in which after the recovery of aliphatic aldehydes from the reaction products, a stream containing unconverted reactants, solvent, unrecovered reaction products, or a combination thereof, is recirculated to the reactor.
